# EUROPEAN PATENT APPLICATION

(11) **EP 4 074 651 A1**
(43) Date of publication of application: **19.10.2022**
(21) Application number: 20899139.8
(22) Date of filing: 18.11.2020
(51) Int. Cl.: C01B 3/00, B01D 61/02, B01D 61/44, C01B 3/22

(54) **HYDROGEN STORAGE METHOD, HYDROGEN GAS PRODUCTION METHOD, AND HYDROGEN GAS PRODUCTION SYSTEM**

(30) Priority: 09.12.2019 JP 2019222351
(71) Applicant: NITTO DENKO CORPORATION, Ibaraki-shi Osaka 567-8680 (JP)
(72) Inventor: MATSUDA Hirokazu, Ibaraki-shi, Osaka 567-8680 (JP); HIRANO Makoto, Ibaraki-shi, Osaka 567-8680 (JP)
(74) Representative: Hoffmann Eitle
(86) International application number: PCT/JP2020/043088
(87) International publication number: WO 2021/117447

(57) **Abstract**

The present invention relates to a hydrogen gas production method, which includes: a first step of concentrating an aqueous solution containing an alkali metal formate; a second step of protonating at least a part of the alkali metal formate by electrodialysis to produce a formic acid; and a third step of decomposing the formic acid to produce a hydrogen gas.

## Description

### TECHNICAL FIELD

The present invention relates to a hydrogen storage method, a hydrogen gas production method, and a hydrogen gas production system.

### BACKGROUND ART

Due to problems such as global warming and fossil fuel depletion, hydrogen energy has been highly expected as next-generation energy. In order to realize a hydrogen energy society, techniques for producing, storing, and utilizing hydrogen are required. However, as for hydrogen storage, there are various problems regarding storage, transportation, safety, cycle, cost and the like.

As hydrogen storage material, various materials such as hydrogen storage alloys, organic hydrides, inorganic hydrides, organic metal complexes, and porous carbon materials has been studied to develop.

The organic hydrides have attracted attention because of advantages such as ease of handling, high hydrogen storage density, and light weight. Some organic hydrides are considered as hazardous substances, and thus may be used as low-concentration solutions. When extracting hydrogen by dehydrogenation reaction, it is necessary to separate and recover hydrogen with high efficiency.

As the organic hydrides, there has been known hydrocarbon compounds such as formic acid, benzene, toluene, biphenyl, naphthalene, cyclohexane, and methylcyclohexane. Since formic acid among these required low energy for dehydrogenation reaction and can be handled easily, the formic acid is considered to be an excellent compound as a hydrogen storage material and is attracting attention.

When formic acid is used as a hydrogen storage material, formic acid is generated in a basic solution by bringing carbon dioxide and hydrogen into contact with each other, or by a reaction of electrochemically reducing carbon dioxide, or the like. However, the reaction is stopped by equilibrium, and only a formic acid solution having a low concentration can be obtained. In order to reduce a transportation cost, it is necessary to obtain a formic acid solution having a high concentration. Further, it is necessary to separate and recover formic acid from a formic acid solution with high efficiency.

Therefore, in Patent Literature 1, for the purpose of performing the production of formic acid by hydrogenation of carbon dioxide, the production of hydrogen by dehydrogenation of formic acid, and the storage and production of hydrogen with high efficiency and high energy efficiency, a method of producing formic acid and/or formate using a catalyst and producing hydrogen from formic acid and/or formate using a catalyst has been studied.

### CITATION LIST

### PATENT LITERATURE

Patent Literature 1: Japanese Patent No. 5812290

### SUMMARY OF INVENTION

### TECHNICAL PROBLEM

The technique described in Patent Literature 1 relates to a catalyst for producing formic acid and producing hydrogen by dehydrogenation of formic acid, and concentration of a hydrogen storage material has not been studied.

Therefore, the present invention provides a hydrogen storage method, a hydrogen gas production method, and a hydrogen gas production system, by which hydrogen can be stored in a state excellent in handling by using an alkali metal formate as a hydrogen storage material, and can be concentrated by a simple method, and a hydrogen gas can be produced with high efficiency.

### SOLUTION TO PROBLEM

Means for solving the above problems are as follows.
[1] A hydrogen gas production method using an alkali metal formate as a hydrogen storage material, the method comprising:
   a first step of concentrating an aqueous solution containing the alkali metal formate;
   a second step of protonating at least a part of the alkali metal formate by electrodialysis to produce formic acid; and
   a third step of decomposing the formic acid to produce a hydrogen gas.
[2] The hydrogen gas production method according to [1], further comprising:
   a step of producing the alkali metal formate in an aqueous solution using carbon dioxide in the presence of an alkali metal salt.
[3] The hydrogen gas production method according to [1] or [2], in which the first step comprises a step of concentrating the aqueous solution containing the alkali metal formate using a separation membrane unit including a reverse osmosis membrane.
[4] The hydrogen gas production method according to any one of [1] to [3], in which the first step comprises a step of distilling off water from the aqueous solution containing the alkali metal formate.
[5] The hydrogen gas production method according to any one of [1] to [4], in which the alkali metal formate is sodium formate.
[6] A hydrogen storage method, comprising: a step of producing an alkali metal formate in an aqueous solution using carbon dioxide in the presence of an alkali metal salt; and a first step of concentrating the aqueous solution containing the alkali metal formate.
[7] The hydrogen storage method according to [6], in which the first step is a step of obtaining a solid of the alkali metal formate by the concentration.
[8] A hydrogen gas production system using an alkali metal formate as a hydrogen storage material, the system comprising:
   a concentration device configured to concentrate an aqueous solution containing the alkali metal formate;
   an electrodialysis device configured to protonate at least a part of the alkali metal formate by electrodialysis to produce formic acid; and
   a formic acid decomposition device configured to decompose the formic acid to produce a hydrogen gas.
[9] The hydrogen gas production system according to [8], further comprising:
   an alkali metal formate production device configured to produce the alkali metal formate in an aqueous solution using carbon dioxide in the presence of an alkali metal salt.

### ADVANTAGEOUS EFFECTS OF INVENTION

According to the present invention, it is possible to provide a hydrogen storage method, a hydrogen gas production method and a hydrogen gas production system by which hydrogen can be stored in a state excellent in handling and can be concentrated by a simple method, and a hydrogen gas can be produced with high efficiency.

### BRIEF DESCRIPTION OF DRAWINGS

[FIG. 1] FIG. 1 is a diagram showing a second step according to an embodiment of the present invention.
[FIG. 2] FIG. 2 is a diagram showing an example of the present invention.
[FIG. 3] FIG. 3 is a diagram showing an example of a hydrogen gas production system according to the embodiment of the present invention.

### DESCRIPTION OF EMBODIMENTS

Hereinafter, embodiments of the present invention will be described in detail.

A hydrogen gas production method according to an embodiment of the present invention is a hydrogen gas production method using an alkali metal formate as a hydrogen storage material. The method includes: a first step of concentrating an aqueous solution containing the alkali metal formate; a second step of protonating at least a part of the alkali metal formate by electrodialysis to produce formic acid; and a third step of decomposing the formic acid to produce a hydrogen gas.

According to the hydrogen gas production method of the embodiment of the present invention, hydrogen can be stored in a state excellent in handling and concentrated by a simple method, and a hydrogen gas can be produced with high efficiency.

### [Alkali metal formate production step]

The hydrogen gas production method according to the embodiment of the present invention may further include a step of producing alkali metal formate in an aqueous solution using carbon dioxide in the presence of an alkali metal salt (alkali metal formate production step).

By the alkali metal formate production step, hydrogen can be stored as an alkali metal formate. The alkali metal formate has a high hydrogen storage density and can be easily handled. Since the alkali metal formate is safe and stable as a chemical substance when the alkali metal formate is used as the hydrogen storage material, the alkali metal formate has an advantage in that the alkali metal formate can be stored for a long period of time. The alkali metal formate aqueous solution produced in this step can be subjected to the first step.

As the alkali metal salt according to the embodiment of the present invention, an inorganic salt of an alkali metal can be used. The alkali metal salt may be used alone or in combination of two or more thereof.

Examples of alkali metal ions constituting a cation moiety of the alkali metal salt include ions of lithium, sodium, potassium, rubidium, and cesium. Among these alkali metal ions, a sodium ion or a potassium ion is preferable.

An anion moiety of the alkali metal salt is not limited as long as the alkali metal formate can be produced. Examples of the anion moiety include a hydroxide ion (OH⁻), a chloride ion (Cl⁻), a bromide ion (Br⁻), an iodide ion (I⁻), a nitrate ion (NO³⁻), a sulfate ion (SO₄²⁻), a phosphate ion (PO₄²⁻), a borate ion (BO₃³⁻), a hydrogen carbonate ion (HCO₃⁻), and a carbonate ion (CO₃²⁻). It is preferable to contain at least one selected from these.

Specific examples of the alkali metal salt include lithium hydroxide, sodium hydroxide, potassium hydroxide, rubidium hydroxide, cesium hydroxide, lithium chloride, sodium chloride, potassium chloride, rubidium chloride, cesium chloride, lithium sulfate, sodium sulfate, potassium sulfate, rubidium sulfate, cesium sulfate, lithium hydrogen carbonate, sodium hydrogen carbonate, potassium hydrogen carbonate, rubidium hydrogen carbonate, cesium hydrogen carbonate, lithium carbonate, sodium carbonate, potassium carbonate, rubidium carbonate, and cesium carbonate. From the viewpoint that by-products are not easily mixed when the alkali metal formate is produced, and the operation after the second step is not complicated, alkali metal hydroxide, alkali metal hydrogen carbonate, or alkali metal carbonate is preferable, and sodium hydroxide, potassium hydroxide, sodium hydrogen carbonate, potassium hydrogen carbonate, sodium carbonate, and potassium carbonate are more preferable.

A content of the alkali metal salt used in the alkali metal formate production step is preferably 0.05 mol/L or more, more preferably 0.1 mol/L or more, and still more preferably 0.2 mol/L or more, from the viewpoint of increasing the production amount of the alkali metal formate. From the viewpoint of resource saving, the content of the alkali metal salt is preferably 20 mol/L or less, more preferably 15 mol/L or less, and still more preferably 10 mol/L or less.

The method for producing alkali metal formate in an aqueous solution using carbon dioxide in the presence of an alkali metal salt is not limited, and may be a method in which carbon dioxide is hydrogenated (allowed to react with hydrogen) in the presence of an alkali metal salt, a method in which carbon dioxide is electrolyzed in the presence of an alkali metal salt, a method in which carbon dioxide is reduced by a photocatalyst in the presence of an alkali metal salt, a method in which carbon dioxide is reduced by a biological technique such as an enzyme in the presence of an alkali metal salt, or a method in which each method is performed in the absence of an alkali metal salt to produce formic acid, and then the formic acid is allowed to react with an alkali metal salt to produce alkali metal formate.

For the hydrogenation reaction of carbon dioxide in the presence of an alkali metal salt, the catalyst to be used is not limited as long as the formic acid can be produced. For example, it is preferable to contain at least one metal element selected from the group consisting of metals belonging to Group 8, Group 9, and Group 10 of a periodic table (hereinafter, simply referred to as a metal element in some cases). Specific examples of the metal element include Fe, Ru, Os, Hs, Co, Rh, Ir, Mt, Ni, Pd, Pt, and Ds. From the viewpoint of catalytic performance, Ru, Ir, Fe and Rh are preferable, and Ru and Ir are more preferable.

The catalyst used in the embodiment of the present invention is preferably soluble in water, an organic solvent, or the like, and more preferably a compound containing a metal element (metal element compound).

Examples of the metal element compound include a salt of a metal element with an inorganic acid such as a hydride salt, an oxide salt, a halide salt (such as a chloride salt), a hydroxide salt, a carbonate salt, a hydrogen carbonate salt, a sulfate salt, a nitrate salt, a phosphate salt, a borate salt, a harate salt, a perharate salt, a harite salt, a hypoharite salt, and a thiocyanate salt; a salt of a metal element with an organic acid such as an alkoxide salt, a carboxylate salt (such as an acetate salt and a (meth)acrylate salt), and a sulfonate salt (such as a trifluoromethanesulfonate salt); a salt of a metal element with an organic base such as an amide salt, a sulfonamide salt, and a sulfonimide salt (such as a bis(trifluoromethanesulfonyl)imide salt); a complex salt such as an acetylacetone salt, a hexafluoroacetylacetone salt, a porphyrin salt, a phthalocyanine salt, and a cyclopentadiene salt; complexes or salts containing one or more of a nitrogen compound containing a chain amine, a cyclic amine, an aromatic amine, and the like, a phosphorus compound, a compound containing phosphorus and nitrogen, a sulfur compound, carbon monoxide, carbon dioxide, water, and the like. These compounds may be either a hydrate or an anhydride, and are not limited. Among these, a halide salt, a complex containing a phosphorus compound, a complex containing a nitrogen compound, and a complex or salt containing a compound containing phosphorus and nitrogen are preferable, from the viewpoint of further enhancing the efficiency of producing formic acid.

These may be used alone or in combination.

As the metal element compound, a commercially available metal element compound can be used, or a metal element compound produced by a known method or the like can also be used. As the known method, for example, a method described in Japanese Patent No. 5896539, or a method described in Chem.Rev. 2017, 117 and 9804-9838, Chem.Rev.2018, 118, 372-433 can be used.

An amount of the catalyst to be used is not limited as long as formic acid or alkali metal formate can be produced. When a metal element compound is used as the catalyst, the amount of the metal element compound to be used is preferably 0.1 µmol or more, more preferably 0.5 µmol or more, and still more preferably 1 µmol or more with respect to 1 L of the solvent in order to sufficiently express the catalytic function. From the viewpoint of cost, the amount is preferably 1 mol or less, more preferably 10 mmol or less, and still more preferably 1 mmol or less. When two or more of the metal element compounds are used, a total amount of the metal element compounds to be used may be within the above range.

In the embodiment of the present invention, the solvent used for producing formic acid or alkali metal formate is not limited, and water, ethylene glycol, polyethylene glycol, glycerin, methanol, ethanol, propanol, pentanol, or the like can be used. More preferably, water, ethylene glycol, polyethylene glycol and glycerin can be used, and still more preferably water can be used. Alternatively, formic acid may be produced using a mixed solvent of water and a solvent miscible with water, and then the solvent miscible with water may be distilled off to form an aqueous solution of formic acid or of an alkali metal formate.

A concentration of the alkali metal formate produced in the alkali metal formate production step is preferably 0.01 mol/L or more, more preferably 0.05 mol/L or more, and still more preferably 0.1 mol/L or more, from the viewpoint of the concentration efficiency in the first step. From the viewpoint of preventing a long time required for the production process of formic acid or alkali metal formate, the concentration is preferably 10 mol/L or less, more preferably 5 mol/L or less, and still more preferably 3 mol/L or less.

### [First step]

The first step is a step of concentrating an aqueous solution containing an alkali metal formate.

The first step may include a step of concentrating the aqueous solution containing the alkali metal formate using a separation membrane unit having a reverse osmosis membrane (concentration step). The first step may further include a step of distilling off water from the aqueous solution containing the alkali metal formate (distillation step). The first step may include either the concentration step or the distillation step, and may include both of the concentration step and the distillation step.

Neither an order nor the number of times for the concentration step and the distillation step is limited. For example, the concentration step and the distillation step may be included in order, and the distillation step, the concentration step, and the distillation step may be included in order. In a region where the concentration of the alkali metal formate is low, the energy required for the distillation step tends to increase, and therefore, it is preferable to include the concentration step and the distillation step in order, from the viewpoint of production efficiency.

The first step is advantageous in that the aqueous solution containing the alkali metal formate is concentrated and the volume is reduced, so that the cost of transportation and storage is reduced and the handling is excellent. Therefore, the alkali metal formate aqueous solution may be concentrated until a solid of the alkali metal formate is precipitated. The precipitated alkali metal formate may be dried.

The first step may be a step of obtaining a solid of an alkali metal formate from the aqueous solution containing the alkali metal formate.

### (Concentration step)

The concentration step is a step of concentrating an aqueous solution containing alkali metal formate using a separation membrane unit provided with a reverse osmosis membrane.

A degree of concentration in the concentration step can be appropriately selected. A concentration of the alkali metal formate in the alkali metal formate aqueous solution after concentration in the concentration step is not limited as long as the concentration is suitable for the subsequent operation, but is preferably 0.1 mol/L or more, more preferably 0.2 mol/L or more, and still more preferably 0.5 mol/L or more, from the viewpoint of energy efficiency in the distillation step. From the viewpoint of preventing problems in the concentration step due to precipitation of the alkali metal formate, the concentration is preferably equal to or lower than a saturation concentration of the alkali metal formate, more preferably equal to or lower than 7 mol/L, and still more preferably equal to or lower than 5 mol/L.

The separation membrane unit according to the embodiment of the present invention includes a reverse osmosis membrane (RO membrane).

The separation membrane unit may be a unit in which a reverse osmosis membrane is housed in a housing, and examples of the form thereof include a flat membrane plate frame type, a pleated type, and a spiral type.

The reverse osmosis membrane is not limited as long as the reverse osmosis membrane is difficult to permeate formic acid ions and alkali metal ions and can concentrate the alkali metal formate aqueous solution, and may be a reverse osmosis membrane (RO membrane), a nano filtration membrane (NF membrane), a micro filtration membrane (MF membrane), or an ultra filtration membrane (UF membrane), but an RO membrane or an NF membrane is preferably used, from the viewpoint of the size of a pore diameter.

The pore diameter of the reverse osmosis membrane is preferably 1 Å or more, more preferably 2 Å or more, and still more preferably 5 Å or more, from the viewpoint of a permeation rate of the aqueous solution. From the viewpoint of a catalyst recovery rate, the pore diameter is preferably 50 Å or less, more preferably 20 Å or less, and still more preferably 10 Å or less.

As the reverse osmosis membrane, a commercially available product can be used, and examples thereof include Nano-SW manufactured by Nitto Denko Corporation, PRO-XS1 manufactured by Nitto Denko Corporation, ESPA-DSF manufactured by Nitto Denko Corporation, CPA7 manufactured by Nitto Denko Corporation, and SWC5-LD manufactured by Nitto Denko Corporation, and ESPA-DSF manufactured by Nitto Denko Corporation or CPA7 manufactured by Nitto Denko Corporation is preferably used.

The concentration step can be performed, for example, using a separation device equipped with a pressure-resistant container under normal pressure or pressure. The pressure in the second step can be adjusted by introducing an inert gas such as nitrogen gas into the pressure-resistant container from a cylinder connected to the pressure-resistant container.

The pressure in the concentration step is more preferably 0.1 MPa or more, and still more preferably 0.3 MPa or more, from the viewpoint of a permeation rate of the solution. From the viewpoint of energy cost due to membrane separation, the pressure is preferably 10.0 MPa or less, more preferably 8 MPa or less, and still more preferably 6 MPa or less.

### (Distillation step)

The degree of concentration in the distillation step can be appropriately selected. The concentration of the alkali metal formate in the alkali metal formate aqueous solution concentrated in the distillation step is not limited as long as the concentration is suitable for the subsequent operation. For example, the concentration is preferably 1 mol/L or more, more preferably 3 mol/L or more, and still more preferably 5 mol/L or more.

The water may be distilled off until the alkali metal formate is precipitated, or the water may be evaporated to dryness until the alkali metal formate is obtained as a solid.

When concentrating the formic acid aqueous solution, a separation and concentration by distillation is difficult to be performed or require a large amount of energy because formic acid and water are azeotropic, but separation of water and the alkali metal formate by distillation of water is facilitated by using the alkali metal formate as the hydrogen storage material, and the alkali metal formate can be obtained as a high-concentration aqueous solution or solid.

In the case where the alkali metal formate is a solid, the cost of transportation and storage is further reduced, and the alkali metal formate can be used as a hydrogen storage material which is more excellent in handling.

As a method of distilling off water, a known method can be used, and for example, water may be distilled off using a known device such as a rotary evaporator or a distillation system, and a degree of pressure reduction and the temperature at this time can be appropriately selected depending on the purpose.

The pressure in the distillation step is preferably normal pressure or less, more preferably 500 mmHg or less, and still more preferably 300 mmHg or less, from the viewpoint of lowering the distillation temperature.

When a solid of alkali metal formate is obtained, the precipitated solid may be dried to dryness. The drying is preferably carried out by using one kind or two or more kinds of operations selected from air blowing, heating, and decompression in combination. Among these, it is preferable to perform drying under reduced pressure or normal pressure while heating at 50°C or more, more preferably 70°C or more, and preferably 200°C or less, and more preferably 170°C or less.

The alkali metal formate is preferably sodium formate because the sodium formate has a low solubility in water and thus is easily precipitated as a solid, and also has a low deliquescence as a solid and is excellent in handling properties.

The alkali metal formate aqueous solution concentrated in the first step may be used in the second step as it is or may be adjusted in concentration by adding pure water as necessary.

When the alkali metal formate is obtained as a solid by concentration, the alkali metal formate can be dissolved in pure water and subjected to the second step.

### [Second step]

The second step is a step of protonating at least a part of the alkali metal formate by electrodialysis to produce formic acid.

In the second step, an alkali metal formate aqueous solution is used.

In the embodiment of the present invention, the aqueous solution containing the alkali metal formate concentrated in the first step is treated using an electrodialysis device, whereby at least a part of the alkali metal formate can be protonated by electrodialysis to produce formic acid.

By subjecting the alkali metal formate solution concentrated in the first step to the second step, a concentrated formic acid solution can be obtained.

In the second step, as described above, the aqueous solution containing the alkali metal formate concentrated in the first step may be used as it is or may be adjusted in concentration by adding pure water as necessary. In addition, an aqueous solution in which the alkali metal formate evaporated to dryness in the first step is dissolved in pure water may be used.

The concentration of the alkali metal formate in the alkali metal formate aqueous solution used for electrodialysis is preferably 0.5 mol/L or more, more preferably 1.0 mol/L or more, and still more preferably 1.5 mol/L or more, from the viewpoint of dialysis efficiency, and is preferably equal to or less than the saturation concentration of the alkali metal formate, more preferably equal to or less than 10 mol/L, and still more preferably equal to or less than 7 mol/L.

In the embodiment of the present invention, a ratio at which the alkali metal formate is protonated in the second step is preferably 10% or more, more preferably 20% or more, and still more preferably 30% or more, with respect to an initial molar amount of the alkali metal formate in the alkali metal formate aqueous solution, from the viewpoint of a formic acid decomposition rate in a third step.

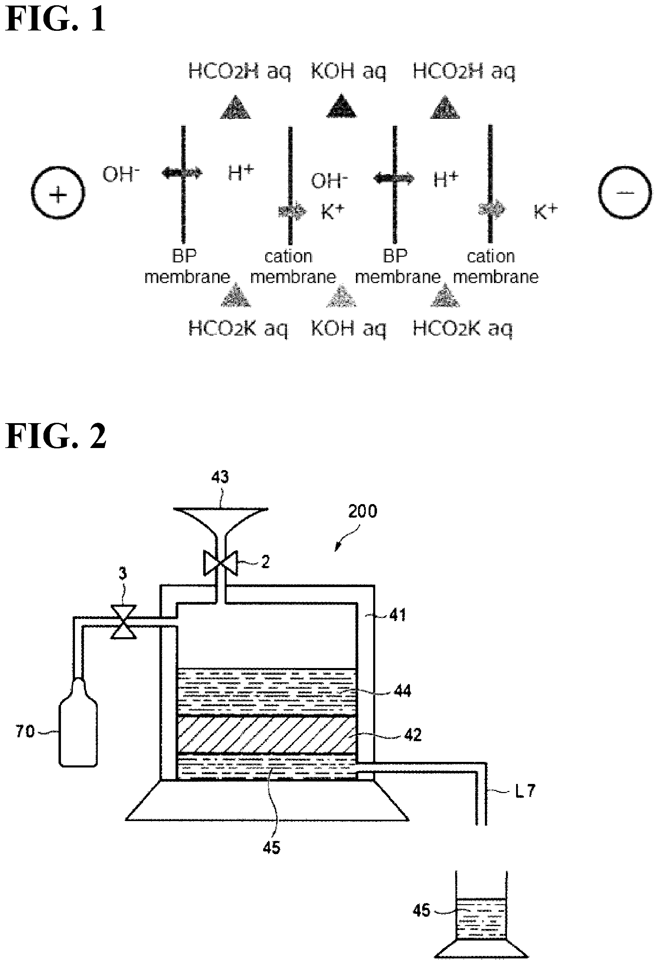

FIG. 1 is a schematic diagram showing an example of the electrodialysis device. The electrodialysis device shown in FIG. 1 includes a plurality of bipolar membranes and a plurality of cation exchange membranes, the bipolar membranes and the cation exchange membranes are alternately disposed between an anode and a cathode, a salt cell is formed between each of the bipolar membranes and the cation exchange membranes disposed on a cathode side thereof, an alkali cell is formed between each of the bipolar membranes and the cation exchange membranes disposed on an anode side thereof, and an organic acid salt aqueous solution is circulated into the salt cell while energizing, so that the alkali metal formate circulated into the salt cell is converted into formic acid while generating alkali metal hydroxide in the alkali cell.

In the second step, the alkali metal formate can be protonated by a simple method to obtain a formic acid solution, and the formic acid solution can be subjected to the third step. When hydrogen is obtained from the alkali metal formate, it is preferable that a part or all of the solution is protonated to make the solution acidic. This is because protons are required when hydrogen is produced from a hydride complex of a formic acid decomposition catalyst metal, which is an intermediate product of formic acid decomposition.

### [Third step]

The third step is a step of decomposing formic acid to produce a hydrogen gas.

In the third step, the formic acid solution obtained in the second step can be used.

The reaction for decomposing formic acid to produce a hydrogen gas may be a reaction for producing a mixed gas containing hydrogen and carbon dioxide from formic acid using a catalyst. The reaction conditions are not limited, and can be appropriately adjusted according to the concentration of the formic acid solution and the type of the catalyst. The reaction conditions can be appropriately changed in the reaction process. The form of a reaction container used for the reaction is not limited.

When a catalyst is used in the third step, the catalyst to be used may be both a homogeneous catalyst and a heterogeneous catalyst.

The catalyst used in the embodiment of the present invention is preferably an organic metal complex containing at least one transition metal selected from iridium, rhodium, ruthenium, cobalt, osmium, nickel, iron, palladium, platinum, and gold, or a salt of the complexes, and more preferably an organic metal complex containing iridium.

In the transition metal-containing organic metal complex (transition metal complex), a counter ion thereof is not limited, and examples of the anion include a hexafluorophosphate ion (PF₆⁻), a tetrafluoroborate ion (BF₄⁻), a hydroxide ion (OH⁻), an acetate ion, a carbonate ion, a phosphate ion, a sulfate ion, a nitrate ion, a halide ion (for example, a fluoride ion (F⁻), a chloride ion (Cl⁻), a bromide ion (Br⁻), and an iodide ion (I⁻)), a hypoharite ion (for example, a hypofluorite ion, a hypochlorite ion, a hypobromite ion, and a hypoiodite ion), a harite ion (for example, a fluorite ion, a chlorite ion, a bromite ion, and an iodite ion), a harate ion (for example, a fluorate ion, a chlorate ion, a bromate ion, and an iodate ion), a perharate ion (for example, a perfluorate ion, a perchlorate ion, a perbromate ion, and a periodate ion), a trifluoromethanesulfonate ion (OSO₂CF₃⁻), and a tetrakispentafluorophenylborate ion (B(C₆F₅)₄).

As the catalyst used in the embodiment of the present invention, a commercially available catalyst can be used, and a catalyst produced by a known method or the like can also be used. As the known method, for example, a method described in JP-A-2018-114495, a method described in Yuichiro Himeda; Nobuko Onozawa-Komatsuzaki; Satoru Miyazawa; Hideki Sugihara; Takuji Hirose; Kazuyuki Kasuga.Chem.Eur.J.2008,14,11076-11081 can be used.

An amount of the catalyst to be used is not limited as long as hydrogen can be produced. From the viewpoint of the rate of the dehydrogenation reaction, the amount is preferably 0.00035 mass% or more, more preferably 0.0035 mass% or more, and still more preferably 0.035 mass% or more, with respect to the solvent of the formic acid solution. The amount of the catalyst to be used is preferably 10 mass% or less, more preferably 5 mass% or less, and still more preferably 3 mass% or less, with respect to the solvent of the formic acid solution, from the viewpoint of durability of the catalyst.

When two or more catalysts are used, a total amount of the catalysts to be used may be within the above range.

In the third step according to the embodiment of the present invention, a solvent may be used. The solvent is preferably a solvent that dissolves the catalyst and becomes uniform, and is not limited, but water, ethylene glycol, polyethylene glycol, glycerin, methanol, ethanol, propanol, pentanol, tetrahydrofuran, dimethylformamide, and the like can be used, and more preferably water, ethylene glycol, polyethylene glycol, glycerin, and even more preferably water can be used.

A reaction temperature is not limited, but is preferably 50°C or higher, more preferably 55°C or higher, and still more preferably 60°C or higher, in order to allow the reaction to proceed efficiently. From the viewpoint of energy efficiency, the temperature is preferably 200°C or lower, more preferably 100°C or lower, and still more preferably 90°C or lower.

The reaction time is not limited, but is, for example, preferably 0.5 hours or more, more preferably 1 hour or more, and still more preferably 2 hours or more, from the viewpoint of ensuring a sufficient hydrogen production amount. From the viewpoint of cost, the time is preferably 24 hours or less, more preferably 12 hours or less, and still more preferably 6 hours or less.

The pressure in the reaction is not limited, but is, for example, preferably 0.1 MPa or more from the viewpoint of ensuring a sufficient hydrogen production amount. From the viewpoint of durability of a hydrogen storage tank, the pressure is preferably 100 MPa or less, more preferably 85 MPa or less, and still more preferably 70 MPa or less.

The method of introducing the solution, the catalyst, the solvent, and the like to be used for the reaction which are obtained in the second step into the reaction container is not limited, but all the raw materials and the like may be introduced collectively, some or all the raw materials may be introduced stepwise, or some or all the raw materials may be introduced continuously. Alternatively, an introduction method in which these methods are combined may be used.

The mixed gas produced in the third step can be separated into a gas containing a hydrogen gas and carbon dioxide.

The purification of the mixed gas is not limited, and examples thereof include purification by a gas separation membrane, gas-liquid separation, a pressure swing adsorption (PSA) method, and the like.

### [Hydrogen storage method]

A hydrogen storage method according to the embodiment of the present invention includes: a step of producing alkali metal formate in an aqueous solution using carbon dioxide in the presence of an alkali metal salt; and a first step of concentrating the aqueous solution containing the alkali metal formate.

In the hydrogen storage method according to the embodiment of the present invention, the first step may be a step of obtaining a solid of alkali metal formate by the concentration.

The step of producing the alkali metal formate and the first step in the hydrogen storage method according to the embodiment of the present invention are the same as those described above in the hydrogen gas production method.

### [Hydrogen gas production system]

A hydrogen gas production system according to an embodiment of the present invention is a hydrogen gas production system using an alkali metal formate as a hydrogen storage material, and includes: a concentration device that concentrate an aqueous solution containing the alkali metal formate, an electrodialysis device that protonates at least a part of the alkali metal formate by electrodialysis to produce formic acid, and a formic acid decomposition device that decomposes the formic acid to produce a hydrogen gas.

The hydrogen gas production system according to the embodiment of the present invention may include an alkali metal formate production device that produces an alkali metal formate in an aqueous solution using carbon dioxide in the presence of an alkali metal salt.

The hydrogen gas production system according to the embodiment of the present invention may include a concentration device 20, an electrodialysis device 30, and a formic acid decomposition device 40. Products obtained by each device may be supplied to other devices after transportation or storage.

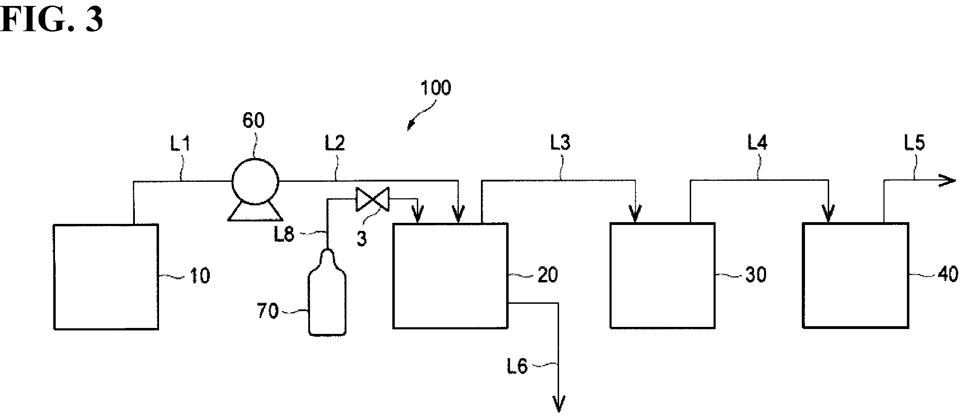

FIG. 3 is a diagram showing an example of the hydrogen gas production system according to the embodiment of the present invention.

A hydrogen gas production system 100 shown in FIG. 3 includes the concentration device 20, the electrodialysis device 30, and the formic acid decomposition device 40, and may further include an alkali metal formate production device 10, a liquid feed pump 60 that feeds an alkali metal formate solution to the concentration device 20, and a cylinder 70 that adjusts a pressure in the concentration device 20. The pressure can be adjusted by a valve 3 provided in a flow path L8.

The hydrogen gas production system 100 shown in FIG. 3 may include a flow path L1 through which the alkali metal formate solution is caused to flow through the liquid feed pump 60, a flow path L2 through which the alkali metal formate solution is supplied from the liquid feed pump 60 to a reactor provided in the concentration device 20, a flow path L3 through which the alkali metal formate solution concentrated by the concentration device 20 is supplied to the electrodialysis device 30, a flow path L4 through which the formic acid solution obtained by electrodialysis is supplied to the formic acid decomposition device 40, and a flow path L5 through which a hydrogen gas generated by decomposition of formic acid is recovered. A flow path L6 through which water, a permeated liquid, and the like are discharged by the concentration device 20 may be provided. Each flow path may include a valve.

According to the present embodiment, it is possible to provide a hydrogen storage method, a hydrogen gas production method, and a hydrogen gas production system, by which hydrogen can be stored in a state excellent in handling and a hydrogen gas can be produced with high efficiency by concentrating the alkali metal formate solution by a simple method.

### [Example]

Hereinafter, the present invention will be described in detail with reference to Examples, but the present invention is not limited to these Examples.

### <Concentration of potassium formate by RO Membran>

### [Example 1]

330 mL of a 2.5 mass% potassium formate aqueous solution was prepared.

As shown in FIG. 2, the concentration step was performed using a separation device 200 provided with a pressure-resistant container 41. ESPA-DSF (manufactured by Nitto Denko Corporation) was installed as an RO membrane 42 in a lower portion of the pressure-resistant container 41 to which a nitrogen cylinder 70 was connected. 330mL of the potassium formate aqueous solution was put from a liquid inlet 43 of the pressure-resistant container 41, and a valve 2 of the liquid inlet 43 was closed. The valve 3 of the nitrogen cylinder 70 was opened, and a pressure was applied with a nitrogen gas of 4 MPa to extrude the solution through the RO membrane 42.

When 130 mL of the liquid was permeated, the pressure was released, and the test was completed.

The separation device 200 may include the flow path L7 through which the permeated liquid was recovered.

The concentration of potassium formate in the liquid (permeated liquid 45) that permeated the RO membrane 42 and the concentration of potassium formate in the liquid (residual liquid) 44 that did not permeate the RO membrane 42 were measured to confirm whether potassium formate could be concentrated. The permeated liquid 45 can be recovered by the flow path L7.

### [Example 2]

An experiment was performed in the same manner as in Example 1 except that the RO membrane was changed from ESPA-DSF to CPA7 (manufactured by Nitto Denko Corporation).

The results of Examples 1 and 2 are shown in Table 1.

### [Table 1]

**Table 1**

| | Membrane type | Test condition | Aqueous solution | Concentration of potassium formate (mass%) | Concentration of potassium formate (mol/L%) |
|---|---|---|---|---|---|
| Example 1 | ESPA-DSF | Undiluted solution 330ml Permeated liquid 130ml Time 176 minutes Pressure 4MPa | Undiluted solution | 2.5 | 0.30 |
| | | | Permeated liquid | 0.9 | 0.11 |
| | | | Residual liquid | 3.8 | 0.45 |
| Example 2 | CPA7 | Undiluted solution 330ml Permeated liquid 130ml Time 163 minutes Pressure 4MPa | Undiluted solution | 2.5 | 0.30 |
| | | | Permeated liquid | 0.3 | 0.04 |
| | | | Residual liquid | 3.8 | 0.45 |

As described above, the potassium formate aqueous solution could be concentrated by a first step. By the first step, an aqueous solution of an alkali metal salt other than potassium can also be concentrated in the same manner.

### <Concentration of alkali formate solution using evaporator>

### [Example 3]

10 g of potassium formate and 90 g of ion-exchanged water were placed in a 200 mL eggplant flask to dissolve potassium formate. Water was volatilized using a water bath heated to 70°C and an evaporator. After 50 minutes, when the content became 12 g, the evaporator was stopped, and the aqueous solution remaining in the eggplant flask was immersed in ice water. Then, a powder was produced. The powder was transferred to a petri dish formed of Teflon (registered trademark) and dried in an oven at 100°C for 2 hours. The dried powder was 6.3 g, and a recovery rate after concentration was 63%.

### [Example 4]

In a 200 mL eggplant flask, 10 g of sodium formate and 90 g of ion-exchanged water were placed, and sodium formate was dissolved. Water was volatilized using a water bath heated to 70°C and an evaporator. After 30 minutes, when the content became 15 g, the powder was produced, and the evaporator was stopped. The powder transferred to a petri dish formed of teflon (registered trademark) and dried in an oven at 100°C for 2 hours. the dried powder was 10.0 g, and a recovery rate after concentration was 100%.

As described above, water could be distilled off from the alkali metal formate aqueous solution in the first step, and the alkali metal formate could be recovered with a high yield.

### <Concentration of alkali formate solution by electrodialysis>

As an electrodialysis device, Ashrizer EX3B manufactured by Astum Co., Ltd. was used.

### [Example 5]

Into a base tank, 500 mL of a 1 mol/L potassium hydroxide aqueous solution was placed.

Into a salt tank, 492 mL of 5 mass% potassium formate aqueous solution was charged.

When the electrodialysis device was started, the voltage was 20.4 V, and the current was 4.41 A.

As the voltage gradually increased, the current decreased, and the dialysis was completed after 15 minutes. At this time, the voltage was 28.0 V, and the current was 3.28 A. After completion of dialysis, the amount of the solution (salt solution) in the salt tank was 468 mL, and the amount of the solution (base solution) in the base tank was 524 mL.

When a formic acid concentration of the salt solution after completion of dialysis was titrated with 0.05 mol/L sodium hydroxide aqueous solution, it was found that 0.58 mol/L of formic acid was generated, and 92.4% of potassium formate was protonated with respect to an initial molar amount of potassium formate.

As the base solution, maleic acid was used as an external standard, heavy water was used as a heavy solvent, and the amount of potassium formate was quantified by ¹H-NMR. As a result, it was found that 5% of a formate anion (HCO₂⁻) was transferred to a base solution side with respect to the initial molar amount of potassium formate.

### [Example 6]

Into a base tank, 522 mL of a 1 mol/L potassium hydroxide aqueous solution was placed.

Into a salt tank, 480 mL of a 10 mass% potassium formate aqueous solution was added.

When an electrodialysis device was started, the voltage was 19.3 V, and the current was 4.41 A.

As the voltage gradually increased, the current decreased, and the dialysis was completed after 25 minutes. At this time, the voltage was 28.0 V, and the current was 4.16 A. After completion of dialysis, the amount of the solution (salt solution) in the salt tank was 432 mL, and the amount of the solution (base solution) in the base tank was 560 mL.

When a formic acid concentration of the salt solution after completion of dialysis was titrated with a 0.05 mol/L sodium hydroxide aqueous solution, it was found that 1.23 mol/L of formic acid was generated, and 88.7% of potassium formate was protonated with respect to an initial molar amount of potassium formate.

As the base solution, maleic acid was used as an external standard, heavy water was used as a heavy solvent, and the amount of potassium formate was quantified by ¹H-NMR. As a result, it was found that 6% of a formate anion (HCO₂⁻) was transferred to a base solution side with respect to the initial molar amount of potassium formate.

### [Example 7]

Into a base tank, 502 mL of a 1 mol/L aqueous potassium hydroxide aqueous solution was placed.

Into a salt tank, 428 mL of a 30 mass% potassium formate aqueous solution was added.

When an electrodialysis device was started, the voltage was 19.4 V, and the current was 4.41 A.

The voltage gradually increased, and the dialysis was completed after 70 minutes. At this time, the voltage was 22.1 V, and the current was 4.41 A. After completion of dialysis, the amount of the solution (salt solution) in the salt tank was 342 mL, and the amount of the solution (base solution) in the base tank was 584 mL.

When a formic acid concentration of the salt solution after completion of dialysis was titrated with a 0.05 mol/L sodium hydroxide aqueous solution, it was found that 3.92 mol/L of formic acid was generated, and 86.4% of potassium formate was protonated with respect to an initial molar amount of potassium formate.

As the base solution, maleic acid was used as an external standard, heavy water was used as a heavy solvent, and the amount of potassium formate was quantified by ¹H-NMR. As a result, it was found that 7% of a formate anion (HCO₂⁻) was transferred to a base solution side with respect to the initial molar amount of potassium formate.

### [Example 8]

Into a base tank, 502 mL of a 1 mol/L aqueous potassium hydroxide aqueous solution was placed.

Into a salt tank, 378 mL of a 50 mass% potassium formate aqueous solution was added.

When an electrodialysis device was started, the voltage was 21.3 V, and the current was 4.41 A.

The voltage gradually increased, and the dialysis was completed after 140 minutes. At this time, the voltage was 27.0 V, and the current was 4.41 A. After completion of dialysis, the amount of the solution (salt solution) in the salt tank was 258 mL, and the amount of the solution (base solution) in the base tank was 622 mL.

When a formic acid concentration of the salt solution after completion of dialysis was titrated with a 0.05 mol/L sodium hydroxide aqueous solution, it was found that 8.21 mol/L of formic acid was generated, and 71.0% of potassium formate was protonated with respect to an initial molar amount of potassium formate.

As the base solution, maleic acid was used as an external standard, heavy water was used as a heavy solvent, and the amount of potassium formate was quantified by ¹H-NMR. As a result, it was found that 15% of a formate anion (HCO₂⁻) was transferred to a base solution side with respect to the initial molar amount of potassium formate.

The results of Examples 5 to 8 are shown in Table 2.

In the table, a formic acid loss rate is a value calculated as a percentage of the molar amount of the formate detected in the base tank by ¹H-NMR with respect to the molar amount of the formate initially added into the salt tank. The solution formic acid concentration is a concentration of formic acid in the salt tank.

An amount of electric power was calculated by reading changes in voltage and current displayed on the device and integrating the amount of electric power (kWh).

### [Table 2]

**Table 2**

| | | Example 5 | Example 6 | Example 7 | Example 8 |
|---|---|---|---|---|---|
| Concentration of potassium formate | | 0.60 mol/L 5 mass% | 1.25 mol/L 10 mass% | 4.17 mol/L 30 mass% | 7.93 mol/L 50 mass% |
| Initial value | pH | 8 | 8.2 | 8.9 | 9.5 |
| After dialysis | pH | 1.7 | 1.2 | 0.5 | <0.0 |
| Dialysis result | Dialysis time (min) | 15 | 25 | 70 | 140 |
| | Protonation rate (%) | 92.4 | 88.7 | 86.4 | 71.0 |
| | Formic acid production amount (g) | 12.5 | 24.4 | 70.9 | 97.4 |
| | Formic acid loss rate (%) | 5 | 6 | 7 | 15 |
| | Solution formic acid concentration (mass%) | 2.6 | 5.6 | 17.3 | 34.9 |
| | Amount of electric power (kWh) | 0.024 | 0.041 | 0.104 | 0.248 |

When the amounts of electric power required for electrodialysis in Examples 5 to 8 were compared, it was found that the amount of electric power required for producing 1 g of formic acid was the lowest in Example 7, and it was possible to perform protonation efficiently.

### [Example 9]

Into a base tank, 500 mL of a 1 mol/L sodium hydroxide aqueous solution was placed.

Into a salt tank, 436 mL of a 24 mass% sodium formate aqueous solution was added.

When an electrodialysis device was started, the voltage was 20.8 V, and the current was 4.41 A.

The voltage gradually increased, and the dialysis was completed after 80 minutes. At this time, the voltage was 24.3 V, and the current was 4.41 A. After completion of dialysis, the amount of the solution (salt solution) in the salt tank was 340 mL, and the amount of the solution (base solution) in the base tank was 582 mL.

When the formic acid concentration of the salt solution after completion of dialysis was titrated with a 0.05 mol/L sodium hydroxide aqueous solution, it was found that 4.31 mol/L formic acid was generated, and 82% of sodium formate was protonated with respect to the initial molar amount of sodium formate.

As the base solution, maleic acid was used as an external standard, heavy water was used as a heavy solvent, and sodium formate was quantified by ¹H-NMR. As a result, it was found that 8% of a formate anion (HCO₂⁻) was transferred to a base solution side with respect to the initial molar amount of sodium formate.

### <Formic acid decomposition>

### (Synthesis of iridium catalyst)

Into a 200 mL eggplant flask, 0.81g of [Cp^{∗}Ir(H₂O)₃](SO₄), 0.82 g of 4,4'-dihydroxy-2,2'-bipyridine, and 60 mL of water were placed. A mixture was stirred in a water bath at 40°C overnight (12 hours).

A black powder was removed by filtration, and the filtrate was concentrated by an evaporator to remove water, thereby obtaining 1.00 g of a yellow powder.

### [Example 10]

In Example 8, formic acid decomposition was performed using a solution obtained by protonating a 50 mass% potassium formate aqueous solution to formic acid by electrodialysis.

25 mL of the solution was placed in a 100 mL eggplant flask, 7.7 mg of the catalyst synthesized above was added into the 100 mL eggplant flask, and the mixture was heated to 60°C to decompose formic acid. The amount of the generated gas was measured by W-NK-0.5B (product number) manufactured by Shinagawa Corporation. The amount of the generated gas after 23.7 hours was 10.65 mL, and a turn over frequency (TOF; a molar amount of hydrogen gas generated per 1 hour with respect to a molar amount of a catalyst) was 1572.

### [Comparative Example 1]

For comparison, a 50 mass% potassium formate aqueous solution (not subjected to electrodialysis) was subjected to formic acid decomposition.

25 mL of the solution was placed in a 100 mL eggplant flask, 7.7 mg of the catalyst synthesized above was added into the 100 mL eggplant flask, and the mixture was heated to 60°C to decompose formic acid. The amount of the generated gas was measured by W-NK-0.5B (product number) manufactured by Shinagawa Corporation. The amount of the generated gas after 3.2 hours was 0.012 mL, and TOF (a molar amount of hydrogen gas generated per 1 hour with respect to a molar amount of a catalyst) was 0.

From the results of Examples 1 to 10, it was revealed that a hydrogen gas can be produced by concentrating an aqueous solution containing alkali metal formate, protonating the alkali metal formate by electrodialysis, and decomposing formic acid with high efficiency. On the other hand, in Comparative Example 1 in which the alkali metal formate was not protonated, a hydrogen gas could not be produced by the decomposition of formic acid.

Although the present invention has been described in detail with reference to specific embodiments, it will be apparent to those skilled in the art that various changes and modifications can be made without departing from the spirit and scope of the present invention.

The present application is based on Japanese Patent Application No. 2019-222351 filed on December 9, 2019, the contents of which are incorporated herein by reference.

### INDUSTRIAL APPLICABILITY

In the hydrogen storage method, the hydrogen gas production method, and the hydrogen gas production system according to the embodiments of the present invention, hydrogen can be stored in a state excellent in handling and concentrated by a simple method, and a hydrogen gas can be produced with high efficiency.

### REFERENCE SIGNS LIST

2, 3 valve
10 Alkali metal formate production device
20 Concentration device
30 Electrodialysis device
40 Formic acid decomposition device
41 Pressure-resistant container
42 RO membrane
43 Liquid inlet
44 Residual liquid
45 Permeated liquid
60 Liquid feed pump
70 Nitrogen cylinder
100 Hydrogen gas production system
200 Separation device
L1, L2, L3, L4, L5, L6, L7, L8 Flow path

## Claims

1. A hydrogen gas production method using an alkali metal formate as a hydrogen storage material, the method comprising:
a first step of concentrating an aqueous solution containing the alkali metal formate;
a second step of protonating at least a part of the alkali metal formate by electrodialysis to produce formic acid; and
a third step of decomposing the formic acid to produce a hydrogen gas.

2. The hydrogen gas production method according to claim 1, further comprising:
a step of producing the alkali metal formate in an aqueous solution using carbon dioxide in the presence of an alkali metal salt.

3. The hydrogen gas production method according to claim 1 or 2, wherein the first step comprises a step of concentrating the aqueous solution containing the alkali metal formate using a separation membrane unit including a reverse osmosis membrane.

4. The hydrogen gas production method according to any one of claims 1 to 3, wherein the first step comprises a step of distilling off water from the aqueous solution containing the alkali metal formate.

5. The hydrogen gas production method according to any one of claims 1 to 4, wherein the alkali metal formate is sodium formate.

6. A hydrogen storage method, comprising: a step of producing an alkali metal formate in an aqueous solution using carbon dioxide in the presence of an alkali metal salt; and a first step of concentrating the aqueous solution containing the alkali metal formate.

7. The hydrogen storage method according to claim 6, wherein the first step is a step of obtaining a solid of the alkali metal formate by the concentration.

8. A hydrogen gas production system using an alkali metal formate as a hydrogen storage material, the system comprising:
a concentration device configured to concentrate an aqueous solution containing the alkali metal formate;
an electrodialysis device configured to protonate at least a part of the alkali metal formate by electrodialysis to produce formic acid; and
a formic acid decomposition device configured to decompose the formic acid to produce a hydrogen gas.

9. The hydrogen gas production system according to claim 8, further comprising: an alkali metal formate production device configured to produce the alkali metal formate in an aqueous solution using carbon dioxide in the presence of an alkali metal salt.
